# EUROPEAN PATENT APPLICATION

(11) **EP 3 744 296 A1**
(43) Date of publication of application: **02.12.2020**
(21) Application number: 19744511.7
(22) Date of filing: 22.01.2019
(51) Int. Cl.: A61F 13/512, A61F 13/15, A61F 13/511

(54) **SHEET, DEVICE FOR MANUFACTURING SHEET, AND METHOD FOR MANUFACTURING SHEET**

(30) Priority: 26.01.2018 JP 2018011867
(71) Applicant: Zuiko Corporation, Settsu-shi Osaka 566-0045 (JP)
(72) Inventor: SHIMADA, Takahiro, Settsu-shi, Osaka 566-0045 (JP); MANABE, Takahito, Settsu-shi, Osaka 566-0045 (JP)
(74) Representative: Müller-Boré & Partner Patentanwälte PartG mbB
(86) International application number: PCT/JP2019/001874
(87) International publication number: WO 2019/146596

(57) **Abstract**

A sheet (1) is used for a disposable wearable article. The sheet (1) is such that a plurality of projections (2) are formed on a single nonwoven fabric. A hole (4) is formed in the apex of the projection (2). The projection (2) includes a holding portion (5), in a periphery of the hole (4) and at a position adjacent to the hole (4), for holding the projection (2) and holding an open state of the hole (4).

## Description

### TECHNICAL FIELD

The present invention relates to a sheet for use in a disposable wearable article such as a disposable diaper and a sanitary napkin, for example, an apparatus for manufacturing the sheet, and a method for manufacturing the sheet.

### BACKGROUND ART

Conventionally, in a disposable wearable article such as a disposable diaper and a sanitary napkin, for example, there is known a sheet (surface sheet) for covering an absorbent member, and for use in a portion in contact with the skin. Such a sheet is formed by nonwoven fabric. The sheet has a function of enhancing the cushioning property and the texture by formation of a plurality of projections. Further, forming a plurality of holes provides a function of enhancing the liquid permeability, thereby moving liquid with a high viscosity to the absorbent member without fail.

For example, Patent Literature 1 discloses a composite sheet as such a sheet for use in a wearable article, and a manufacturing method thereof. The composite sheet is such that a first sheet having a plurality of projections and recesses, and a flat-shaped second sheet are partially joined to each other at a plurality of joint portions. A plurality of holes are formed in the composite sheet. The composite sheet is manufactured as follows. Specifically, the first sheet is supplied to engageable portions of a first roll and a second roll, while rotating the first roll and the second roll each having a mutually engageable concavo-convex shape on an outer circumferential surface thereof. Then, the first sheet is moved away from the engageable portions, while being held on the outer circumferential surface of the first roll. Thereafter, the first sheet is placed over the second sheet and is partially joined to the second sheet. The joined first and second sheets are moved while being held on the outer circumferential surface of the first roll. Thereafter, a plurality of holes are formed by a perforation roll having a perforation portion on an outer circumferential surface thereof.

By the way, if the above-described sheet (surface sheet) for use in a wearable article can be manufactured from a single nonwoven fabric, remarkable cost reduction can be realized.

However, in Patent Literature 1, since the projections formed on the first sheet by the first roll and the second roll cannot be held solely on the first sheet, the projections cannot be continued to be present on the first sheet. The projections are held only after the second sheet is joined to the first sheet. Therefore, in the manufacturing method disclosed in Patent Literature 1, it may not be possible to manufacture a sheet for a disposable wearable article from a single nonwoven fabric.

Further, in the manufacturing method disclosed in Patent Literature 1, the holes formed in the composite sheet are formed by stretching the sheet by the perforation portion of the perforation roll. Therefore, the holes may shrink or may close again after the perforation portion is taken out from the sheet, and it is not possible to form clear holes.

### CITATION LIST

### PATENT LITERATURE

Patent Literature 1: Japanese Patent No. 4863697

### SUMMARY OF INVENTION

An object of the present invention is to form a sheet (surface sheet) for use in a disposable wearable article from a single nonwoven fabric, hold a projection formed on the sheet, and hold an open state of a hole.

The present invention is directed to a sheet for use in a disposable wearable article. The sheet is such that a plurality of projections are formed on a single nonwoven fabric. A hole is formed in an apex of the projection. The projection includes a holding portion, in a periphery of the hole and at a position adjacent to the hole, for holding the projection and holding an open state of the hole.

A sheet manufacturing apparatus according to the present invention is an apparatus for manufacturing a sheet for use in a disposable wearable article from a single nonwoven fabric. The manufacturing apparatus includes: an ultrasonic horn including an application surface for applying ultrasonic vibration; and an anvil including an opposing surface capable of facing the application surface. A plurality of protrusions are formed on the opposing surface. Each of the protrusions includes a top surface located at a distal end of the protrusion, and an adjoining surface that is formed to adjoin the top surface in such a way as to extend toward a base end of the protrusion, while spreading from the top surface to a periphery of the top surface. The manufacturing apparatus is configured to apply ultrasonic vibration with the nonwoven fabric being sandwiched between the application surface and the opposing surface in a state that a plurality of projections are formed on the nonwoven fabric by attracting the nonwoven fabric to the opposing surface where the plurality of protrusions are formed in such a way as to melt a portion of the nonwoven fabric associated with the top surface and form a hole in the projection, and is configured to cool the melted portion in a periphery of the hole in such a way as to form a holding portion, in the periphery of the hole and at a position adjacent to the hole, for holding the projection and holding an open state of the hole.

A sheet manufacturing method according to the present invention is a method for manufacturing a sheet for use in a disposable wearable article from a single nonwoven fabric. The manufacturing method includes: a projection forming process of forming a plurality of projections on the nonwoven fabric; a hole forming process of melting at least a part of an apex of the projection and forming a hole by application of ultrasonic vibration with the nonwoven fabric being sandwiched between an application surface of an ultrasonic horn for applying ultrasonic vibration and an opposing surface of an anvil facing the application surface in a state that the plurality of projections are formed on the nonwoven fabric; and a holding portion forming process of forming a holding portion, in a periphery of the hole and at a position adjacent to the hole, for holding the projection and holding an open state of the hole by cooling, in the periphery of the hole, at least a part of the apex of the projection melted in the hole forming process.

The present invention is able to form a sheet for use in a disposable wearable article from a single nonwoven fabric, hold a projection formed on the sheet, and hold an open state of a hole. Therefore, it is possible to provide the good cushioning property of the sheet, and provide the good liquid permeability of the sheet, while reducing the cost.

### BRIEF DESCRIPTION OF DRAWINGS

FIG. 1 is a perspective view illustrating a sheet according to an embodiment of the present invention.
FIG. 2 is a cross-sectional view taken along the line II-II in FIG. 1.
FIG. 3 is a plan view illustrating the sheet in FIG 1.
FIG. 4 is a schematic front view illustrating a manufacturing apparatus according to the embodiment of the present invention.
FIG. 5 is a plan view enlargedly illustrating a part of an anvil of the manufacturing apparatus in FIG. 4.
FIG. 6 is a front cross-sectional view enlargedly illustrating a protrusion of the anvil of the manufacturing apparatus in FIG. 4.
FIG. 7 is a front cross-sectional view enlargedly illustrating a part of the anvil of the manufacturing apparatus in FIG. 4.
FIG. 8 is a perspective view illustrating a sheet according to a modification of the embodiment.

### DESCRIPTION OF EMBODIMENTS

In the following, an embodiment according to the present invention is described with reference to the drawings. The following embodiment is an example embodying the present invention, and does not intend to limit the technical scope of the present invention.

### [Sheet]

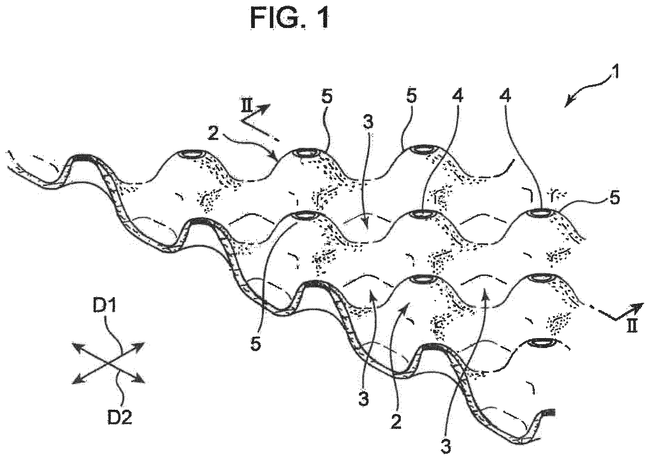

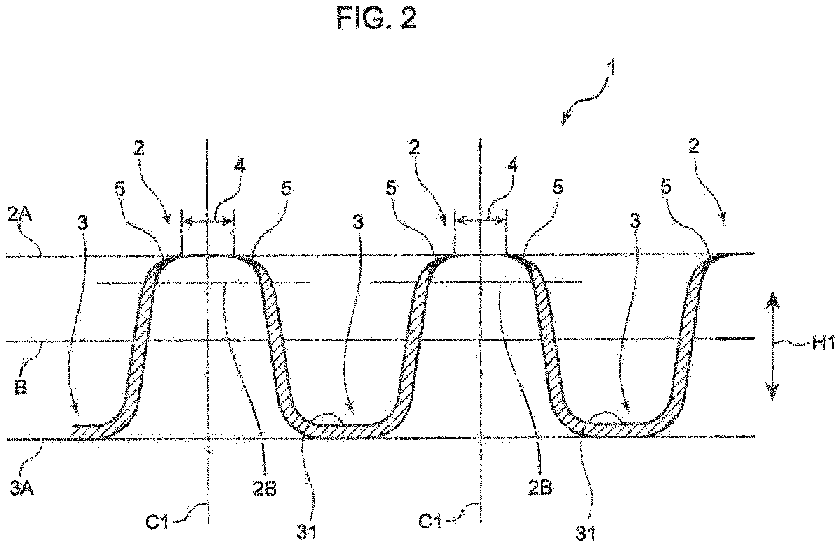

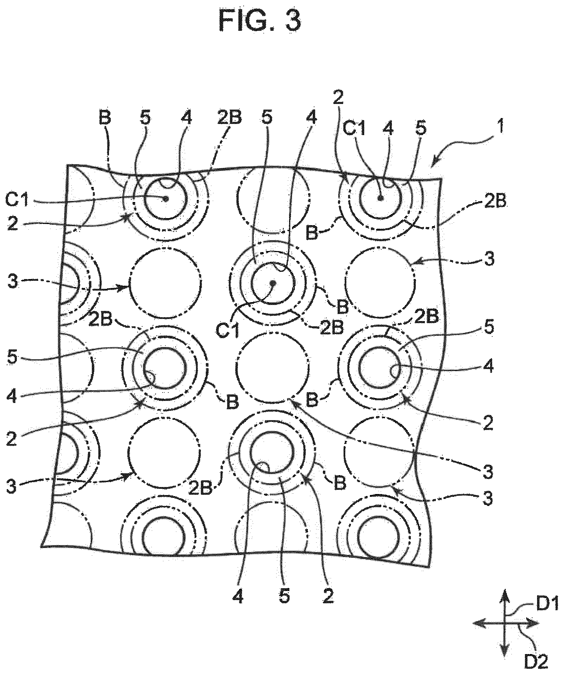

FIG. 1 is a perspective view illustrating a sheet 1 according to an embodiment of the present invention. FIG. 2 is a cross-sectional view taken along the line II-II in FIG. 1. FIG. 3 is a plan view illustrating the sheet 1 in FIG. 1. The sheet 1 according to the present embodiment illustrated in FIGS. 1 to 3 can be used, in a disposable wearable article such as a disposable diaper and a sanitary napkin, for example, as a sheet for covering an absorbent member, and for use in a portion in contact with the skin. However, the usage of the sheet 1 is not limited to the above.

The sheet 1 can be manufactured from a single nonwoven fabric by employing a manufacturing apparatus 10 and a manufacturing method according to the embodiment of the present invention to be described later. As the nonwoven fabric, a nonwoven fabric made of thermoplastic resin such as polypropylene and polyethylene can be exemplified. However, the nonwoven fabric is not limited to the above.

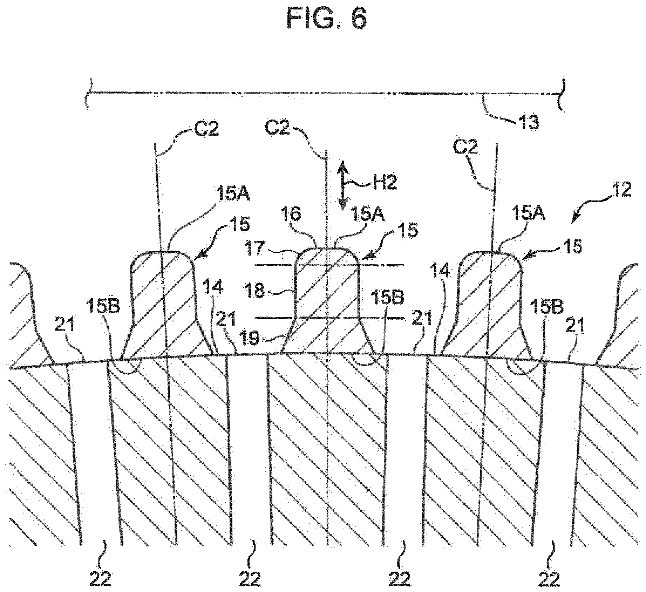

As illustrated in FIGS. 1 to 3, the sheet 1 includes alternately aligned projections 2 and recesses 3. Each of the projections 2 is formed by bulging a part of a single nonwoven fabric in one direction (upward in FIG. 2) of height directions H1 illustrated in FIG. 2 with respect to the recess 3. An overall configuration of the projection 2 can be adjusted by changing the shape of a protrusion 15 (see FIG. 6) of an anvil of the manufacturing apparatus 10 for the sheet 1 to be described later.

In the sheet 1, the plurality of projections 2 are aligned at a predetermined pitch along a first direction D1, and are aligned at a predetermined pitch along a second direction D2 orthogonal to the first direction D1. Likewise, the plurality of recesses 3 are aligned at a predetermined pitch along the first direction D1, and are aligned at a predetermined pitch along the second direction D2 orthogonal to the first direction D1. However, the plurality of projections 2 may not be necessarily aligned at a fixed interval. Likewise, the plurality of recesses 3 may not be necessarily aligned at a fixed interval.

As illustrated in FIG. 2, in the sheet 1, a boundary B between the projection 2 and the recess 3 is located at an intermediate position between an apex 2A of the projection 2 and a bottom portion 3A of the recess 3 in the height direction H1 of the projection 2. Therefore, in the present embodiment, a base portion of the projection 2 is located at the boundary B.

The sheet 1 includes a plurality of holes 4 for enhancing the liquid permeability and the air permeability. Each of the holes 4 is formed in the apex 2A of the associated projection 2. A size and a shape of the hole 4 can be appropriately adjusted by changing a shape of the protrusion 15 of the anvil of the manufacturing apparatus 10 for the sheet 1 to be described later, a time, an amplitude, an applied pressure, and the like of ultrasonic vibration by an ultrasonic horn 11. In the present embodiment, the hole 4 has a circular shape in a plan view illustrated in FIG. 3. However, the shape of the hole 4 is not limited to the circular shape. The shape of the hole 4 can be changed according to the shape of a top surface 16 (see FIG. 6) of the protrusion 15 formed on an outer circumferential surface 14 of the anvil.

The sheet 1 includes a plurality of holding portions 5 for holding the plurality of projections 2. Each of the holding portions 5 is formed on the associated projection 2. The holding portion 5 is formed in the periphery of the hole 4 and at a position adjacent to the hole 4. In the present embodiment illustrated in FIG. 2, the holding portion 5 is formed on a part of the projection 2 on a side of the apex 2A. Specifically, the holding portion 5 is formed in an area from a position 2B (e.g., a position in proximity to the apex 2A) to the hole 4 in the height direction H1 of the projection 2, the position 2B is a position between the apex 2A and the boundary B. Alternatively, the holding portion 5 may be formed in a larger area on the projection 2.

As illustrated in FIG. 3, when the sheet 1 is planarly viewed, the holding portion 5 has an annular shape. The hole 4 is defined by an inner circumference of the holding portion 5.

As illustrated in FIGS. 1 and 2, the holding portion 5 includes a part which approaches a base portion B of the projection 2 in the height direction H1 as a distance from the hole 4 in a direction orthogonal to the height direction H1 increases. Specifically, the holding portion 5 is not only planarly formed on the apex 2A of the projection 2 to simply surround a circumference of the hole 4, but also is stereoscopically formed to spread toward the base portion B of the projection 2 in the height direction H1 of the projection 2. Forming the holding portion 5 to have such a configuration enables to further enhance the effect of holding the projection 2.

A rigidity of the holding portion 5 is higher than a rigidity of a portion other than the holding portion 5, such as a bottom portion 31 of the recess 3. Configuring the sheet 1 in such a way that the rigidity of the holding portion 5 is higher than the rigidity of the portion other than the holding portion 5 enables to hold the projection 2 and hold an open state of the hole 4.

A mass per unit area (basis weight) of the holding portion 5 is greater than a mass per unit area (basis weight) of the portion other than the holding portion 5, such as the bottom portion 31 of the recess 3. Configuring the sheet 1 in such a way that the mass per unit area of the holding portion 5 is greater than the mass per unit area of the portion other than the holding portion 5 enables to hold the projection 2 and hold an open state of the hole 4.

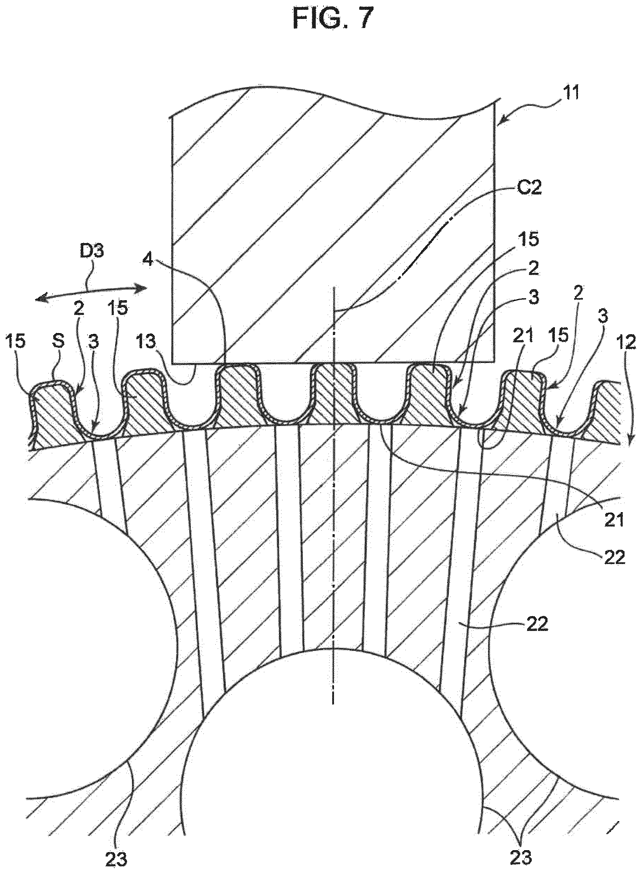

As a method for setting the rigidity of the holding portion 5 higher than the rigidity of the portion other than the holding portion 5, or setting the mass per unit area of the holding portion 5 greater than the mass per unit area of the portion other than the holding portion 5, the manufacturing method for the sheet 1 according to the embodiment to be described later can be exemplified. A nonwoven fabric S being a raw material for the sheet 1 is a sheet-like material in which fibers formed of thermoplastic resin as described above or the like are intertwined without being woven, and has flexibility. In the sheet 1 to be manufactured by the below-described manufacturing method, the hole 4 is formed by melting a part of the nonwoven fabric S by frictional heat by the ultrasonic horn 11, molten matter is moved to the periphery of the hole 4 and a position adjacent to the hole 4 due to a pressure applied by a distal end surface 13 (see FIG. 7) of the ultrasonic horn 11, and the molten matter is cooled and turns into the holding portion 5. In this way, since the holding portion 5 includes a portion such that a part of the nonwoven fabric S is melt and then hardened, the portion turns into a lump in which voids are less, as compared with the nonwoven fabric S before being processed. Consequently, the rigidity of the holding portion 5 is made high, as compared with the portion other than the holding portion 5. Further, since the molten matter of the nonwoven fabric S that was in an area corresponding to the hole 4 flows into the holding portion 5, the mass per unit area of the holding portion 5 becomes greater than that of the portion other than the holding portion 5.

As described above, in the sheet 1 according to the present embodiment, the holding portion 5 formed in the periphery of the hole 4 and at the position adjacent to the hole 4 holds the projection 2, and holds an open state of the hole 4. Therefore, it is possible to form, from a single nonwoven fabric, the sheet 1 for use in a disposable wearable article, and hold the projection 2 formed on the sheet 1 and an open state of the hole 4.

Further, in the sheet 1 according to the present embodiment, the holding portion 5 has a shape such that a position in the height direction H1 approaches the base portion B of the projection 2, as a distance from the hole 4 in a direction orthogonal to the height direction H1 increases. In this way, the holding portion 5 is not only planarly formed to simply surround the circumference of the hole 4, but also is stereoscopically formed to spread toward the base portion B of the projection 2 in the height direction H1 of the projection 2. Thus, the effect of holding the projection 2 by the holding portion 5 can be further enhanced.

Furthermore, the sheet 1 according to the present embodiment has the cushioning property by keeping the concavo-convex shape. In addition, the sheet 1 is able to maintain the high permeability of body fluid by holding an open state of the hole 4.

### [Sheet Manufacturing Apparatus]

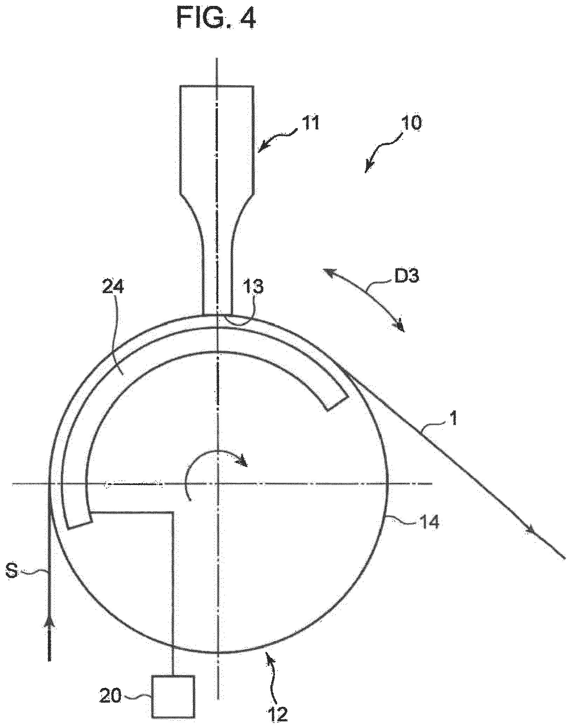

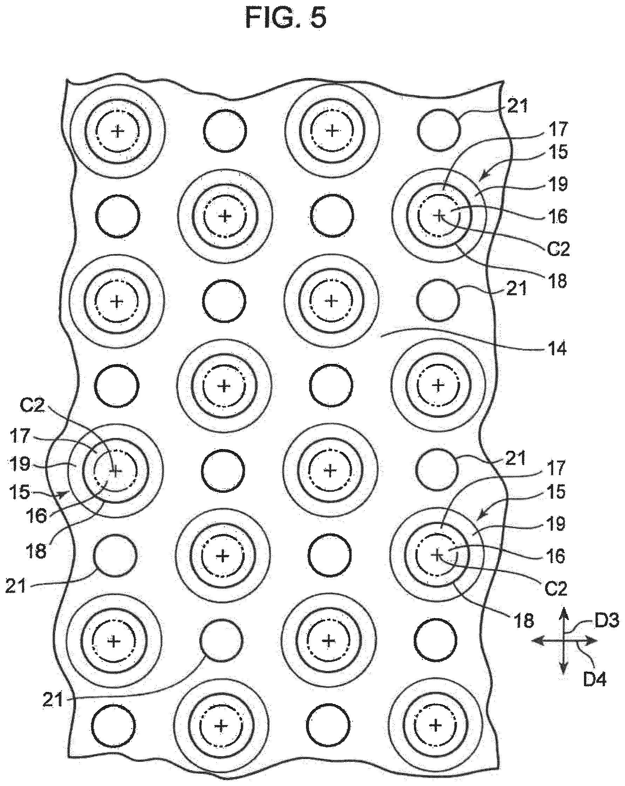

FIG. 4 is a schematic front view illustrating the manufacturing apparatus 10 according to the embodiment of the present invention. FIG. 5 is a plan view enlargedly illustrating a part of an anvil of the manufacturing apparatus 10 in FIG. 4. FIG. 6 is a front cross-sectional view enlargedly illustrating the protrusions 15 of the anvil of the manufacturing apparatus 10 in FIG. 4. FIG. 7 is a front cross-sectional view enlargedly illustrating a part of the anvil of the manufacturing apparatus 10 in FIG. 4.

The manufacturing apparatus 10 according to the present embodiment is an apparatus for manufacturing the above-described sheet 1. As illustrated in FIG. 4, the manufacturing apparatus 10 includes an ultrasonic application device, the anvil, and a suction device 20.

The ultrasonic application device includes the ultrasonic horn 11, an unillustrated ultrasonic oscillator, an unillustrated converter, an unillustrated booster, and the like. The ultrasonic horn 11 has the distal end surface 13 (application surface 13) for applying ultrasonic vibration. In the present embodiment, the distal end surface 13 is constituted of a flat surface. The distal end surface 13 is a flat surface extending in a tangential direction of the outer circumferential surface 14 of a roller 12 to be described later.

The ultrasonic oscillator is electrically connected to the converter, and is configured to input, to the converter, an electric signal having a wavelength in a predetermined range of a frequency output from the ultrasonic oscillator. The converter is incorporated with a piezoelectric element, for example, and converts the electric signal input from the ultrasonic oscillator into mechanical vibration. The booster adjusts the amplitude of the mechanical vibration generated from the converter, and transmits the mechanical vibration to the ultrasonic horn 11. The ultrasonic horn 11 is configured to resonate at a frequency in use.

In the present embodiment, the anvil includes the tubular-shaped roller 12, and a plurality of pins 15 (protrusions 15). The roller 12 is configured to be rotatable in a direction of an arrow illustrated in FIG. 4 by an unillustrated motor. The roller 12 has the outer circumferential surface 14 (opposing surface 14) capable of facing the distal end surface 13 of the ultrasonic horn 11.

As illustrated in FIG. 5, the plurality of pins 15 are formed on the outer circumferential surface 14 of the roller 12. The plurality of pins 15 are formed on the outer circumferential surface 14 at a predetermined pitch. Specifically, the plurality of pins 15 are aligned at a predetermined pitch along a circumferential direction D3 of the outer circumferential surface 14 of the roller 12, and are aligned at a predetermined pitch along a width direction D4 of the outer circumferential surface 14 (axis direction D4 of a rotational axis of the roller 12). However, the plurality of pins 15 can be disposed in any pattern. The plurality of pins 15 may not be necessarily aligned at a fixed interval. The plurality of pins 15 may be aligned in a direction intersecting the circumferential direction D3 or the width direction D4 at any angle, or may not be aligned in a specific direction.

As illustrated in FIGS. 5 and 6, each of the pins 15 is formed to protrude radially outward of the roller 12 from the outer circumferential surface 14 of the roller 12. Each of the pins 15 includes a base end 15B continuing to the outer circumferential surface 14, and a distal end 15A being an end in the protruding direction.

Each of the pins 15 has an outer surface. The outer surface of each of the pins 15 includes a top surface 16, an adjoining surface 17 continuing to the top surface 16, a first lateral surface 18, and a second lateral surface 19. These surfaces 16 to 19 are formed in this order in a height direction H2 of each of the pins 15.

The top surface 16 is a distal end surface located at a distal end in the protruding direction, which is one of the height directions H2 of the pin 15. The top surface 16 is a surface closest to the distal end surface 13 among the outer surfaces of the pin 15, when the pin 15 faces the distal end surface 13 of the ultrasonic horn 11. In other words, the top surface 16 is a surface farthest from the rotational axis of the roller 12 among the outer surfaces of the pin 15. In the embodiment illustrated in FIG. 6, the top surface 16 is constituted of a flat surface formed in an area where the top surface 16 intersects with a centerline C2 of the pin 15 in parallel to the height direction H2.

The adjoining surface 17 is a surface formed to continue to the top surface 16 in such a way as to spread from the top surface 16 to the periphery thereof. The adjoining surface 17 is located far from the distal end surface 13 than the top surface 16, when the top surface 16 faces the distal end surface 13 of the ultrasonic horn 11. In other words, the adjoining surface 17 is located near the rotational axis of the roller 12 than the top surface 16.

As illustrated in FIG. 6, in the present embodiment, the adjoining surface 17 has a shape such that a cross-sectional area of the pin 15 decreases as a distance from the top surface 16 decreases. Specifically, an area of a cross-section of the pin 15 orthogonal to the centerline C2 gradually decreases as a distance from the top surface 16 decreases, in a part of the pin 15 where the adjoining surface 17 is formed. In this example, the shape of the adjoining surface 17 in a cross section including the centerline C2 of the pin 15 includes, as illustrated in FIG. 6, not only a curve, but also a straight line, a combination of a curve and a straight line, and the like. The adjoining surface 17 may not be a continued surface such that the entirety is smooth, and may include a surface with a angular part.

As illustrated in FIG. 6, the application surface 13 has a size such that the entirety of the top surface 16 and the entirety of the adjoining surface 17 are included, when the application surface 13, the top surface 16, and the adjoining surface 17 are viewed in the height direction H2 in a state that the top surface 16 of the protrusion 15 faces the application surface 13 of the ultrasonic horn 11.

The first lateral surface 18 is a surface, which is formed to continue to the adjoining surface 17, located near the base end 15B than the adjoining surface 17. The first lateral surface 18 is a surface that does not have a shape such that a cross-sectional area of the pin 15 decreases as a distance from the top surface 16 decreases, unlike the adjoining surface 17. The first lateral surface 18 is a lateral surface (cylindrical surface) of a cylindrical portion of the pin 15. An area of a cross-section of the pin 15 orthogonal to the centerline C2 is fixed in a part of the pin 15 where the first lateral surface 18 is formed.

The second lateral surface 19 is a surface, which is formed to continue to the first lateral surface 18, located near the base end 15B than the first lateral surface 18. The second lateral surface 19 is continued to the outer circumferential surface 14 of the roller 12. The second lateral surface 19 has a shape such that an area of a cross-section of the pin 15 orthogonal to the centerline C2 gradually decreases, as approaching the first lateral surface 18 from the base end 15B, in a part of the pin 15 where the second lateral surface 19 is formed. Forming the second lateral surface 19 to have a shape as described above allows a part of the nonwoven fabric S to have a smooth curved surface along the second lateral surface 19, when air is sucked through suction ports 21, and the nonwoven fabric S comes into firm contact with the outer circumferential surface 14 and the pins 15, as illustrated in FIG. 7.

As illustrated in FIG. 7, the roller 12 includes a plurality of suction ports 21 opened in the outer circumferential surface 14, a plurality of suction channels 22 respectively communicating with the plurality of suction ports 21, and a plurality of joint channels 23 at which air to be sucked through the plurality of suction channels 22 is merged. The plurality of suction ports 21 are formed in positions between the protrusions 15 adjacent to one another on the outer circumferential surface 14 of the roller 12. Each of the suction channels 22 extends radially inward of the roller 12 from the associated suction port 21. Each of the joint channels 23 extends in the axis direction of the roller 12. The plurality of joint channels 23 are aligned along the circumferential direction D3 of the roller 12. The joint channels 23 communicate with the suction device 20 as a suction source via a suction box 24 (see FIG. 4), which is provided at an end surface of the roller 12 in the axis direction.

The suction device 20 is configured to suck air through the suction ports 21, and attracts the nonwoven fabric S to the outer circumferential surface 14. The suction device 20 communicates with the suction ports 21 in the outer circumferential surface 14 of the roller 12 via the joint channels 23 and the suction channels 22.

In a front view illustrated in FIG. 4, the suction box 24 has an arc shape. The suction box 24 is not formed over the entire periphery of an end surface, which is located at one end of the roller 12 in the axis direction, but is formed at a part of the end surface, which is associated with an area where the nonwoven fabric S is wound on the outer circumferential surface 14 of the roller 12. The suction box 24 is not configured to rotate accompanied by rotation of the roller 12. A relative position between the suction box 24 and the ultrasonic horn 11 does not change. Thus, an area on the outer circumferential surface 14 of the roller 12 within which air is sucked by the suction device 20 is defined.

### [Sheet Manufacturing Method]

Next, the manufacturing method according to the present embodiment is described. The manufacturing method according to the present embodiment is a method for manufacturing the sheet 1 for use in a disposable wearable article from the single nonwoven fabric S. The manufacturing method includes a projection forming process, a hole forming process, and a holding portion forming process.

In the below-described manufacturing method according to the embodiment, the roller 12 is not stopped, and is continuously rotated in the direction of the arrow in FIG. 4 during a period when the projection forming process, the hole forming process, and the holding portion forming process are performed; and a distance between the ultrasonic horn 11 and the pin 15 of the anvil is kept to a predetermined value. However, the manufacturing method according to the present invention is not limited to the method described in the embodiment. For example, the roller 12 may be intermittently rotated during a period when the projection forming process, the hole forming process, and the holding portion forming process are performed. Further, the ultrasonic horn 11 may be configured to be movable toward and away from the outer circumferential surface 14 of the roller 12.

It is possible to define a section within which the projection forming process, the hole forming process, and the holding portion forming process are performed in association with a section within which the suction box 24 is provided. Specifically, as illustrated in FIG. 4, the projection forming process is performed in an area on the upstream side in the rotating direction with respect to an area associated with the distal end surface 13 of the ultrasonic horn 11, in a part associated with the section within which the suction box 24 is provided. The hole forming process is performed in the area associated with the distal end surface 13 of the ultrasonic horn 11, in the part associated with the section within which the suction box 24 is provided. The holding portion forming process is performed in an area on the downstream side in the rotating direction with respect to the area associated with the distal end surface 13 of the ultrasonic horn 11, in the part associated with the section in which the suction box 24 is provided.

The projection forming process is a process of forming a configuration in which projections and recesses are alternately formed on the nonwoven fabric S in advance prior to the hole forming process and the holding portion forming process. In the projection forming process, as illustrated in FIG. 4, the nonwoven fabric S fed to the roller 12 is sucked and attracted to the outer circumferential surface 14 of the roller 12 and the plurality of pins 15 by causing the suction device 20 to suck air within the suction channels 22. Therefore, the nonwoven fabric S turns into a shape along the outer circumferential surface 14 of the roller 12, and the outer surfaces of the plurality of pins 15 formed on the outer circumferential surface 14. Thus, the alternately aligned projections 2 and recesses 3 are formed on the nonwoven fabric S.

The hole forming process is a process of melting a part of the projection 2 and forming the hole 4 by applying ultrasonic vibration in a state that the nonwoven fabric S is sandwiched between the distal end surface 13 of the ultrasonic horn 11, and the outer circumferential surface 14 of the roller 12.

In the hole forming process, the ultrasonic horn 11 applies ultrasonic vibration to the nonwoven fabric S, while applying a predetermined pressure to the nonwoven fabric S. Specifically, ultrasonic vibration transmitted from the above-described booster to the ultrasonic horn 11 is applied to the nonwoven fabric S, which is sandwiched between the distal end surface 13 of the ultrasonic horn 11, and the plurality of pins 15 on the roller 12. Thus, a part of the nonwoven fabric S to which the ultrasonic vibration is applied, specifically, a part associated with each of the top surfaces 16 of the plurality of pins 15 is melted by frictional heat, and molten matter is moved to the periphery, thereby forming the above-described hole 4 in the nonwoven fabric S. Since air suction is also performed in the hole forming process, the projection 2 in which the hole 4 is formed is transferred to the downstream side in the rotating direction of the roller 12, while keeping the shape along the protrusion 15 on the roller 12.

In the present embodiment, manufacturing conditions such as a time, an amplitude, and an applied pressure of ultrasonic vibration by the ultrasonic horn 11 in the hole forming process are set to values capable of forming the hole 4 in the nonwoven fabric S according to a material, a thickness, and the like of the nonwoven fabric S.

The holding portion forming process is a process of forming the holding portion 5 for holding the shape of the projection 2 and the shape of the hole 4, in the periphery of the hole 4 and at the position adjacent to the hole 4 by cooling, in the periphery of the hole 4, a part of the projection 2 melted in the hole forming process.

When a part of the nonwoven fabric S passes through an area where the nonwoven fabric S faces the distal end surface 13 of the ultrasonic horn 11, and the hole 4 is formed, in the succeeding holding forming process, a portion in the periphery of the hole 4 and adjacent to the hole 4 is cooled on the downstream side with respect to the area where the nonwoven fabric S faces the distal end surface 13 of the ultrasonic horn 11, and the above-described holding portion 5 is formed. In the present embodiment, cooling of the above-described portion is performed by a stream of air generated by causing the suction device 20 to suck the surrounding air. Alternatively, the above-described portion may be forcibly cooled by an unillustrated blower or the like.

In the present embodiment, in the projection forming process, the nonwoven fabric S is attracted to the outer circumferential surface 14 of the roller 12 and the pins 15 by sucking air through the suction ports 21. It should be noted that air suction is continuously performed also in the hole forming process and the holding portion forming process.

As described above, in the manufacturing apparatus 10 according to the present embodiment, a portion of the nonwoven fabric S associated with the top surface 16 is melted by ultrasonic vibration, and the hole 4 and the holding portion 5 are formed. Therefore, it is possible to allow the holding portion 5 to hold the projection 2 and hold an open state of the hole 4. Thus, it is possible to form, from a single nonwoven fabric, the sheet 1 for a disposable wearable article in which the projection 2 is held, and an open state of the hole 4 is held.

Further, in the manufacturing apparatus 10 according to the present embodiment, each of the protrusions 15 includes the top surface 16 located at a distal end 15A of the protrusion 15, and the adjoining surface 17 which is formed to continue to the top surface 16 in such a way as to spread from the top surface 16 to the periphery thereof; and the adjoining surface 17 is located near to the base end I5B of the protrusion 15 than the top surface 16. Therefore, when a part of the projection 2 is melted by applying ultrasonic vibration in a state that the nonwoven fabric S is sandwiched between the application surface 13 and the opposing surface 14, and the hole 4 is formed, the melted part is pressed against the top surface 16. Accordingly, the melted part is moved to the periphery of the hole 4 along the adjoining surface 17 which is formed to continue to the top surface 16. The thus moved part is cooled and solidified at a position along the adjoining surface 17. Consequently, the holding portion 5 is not only planarly formed to simply surround the periphery of the hole 4, but also is stereoscopically formed to spread toward the base portion B of the projection 2 (bottom portion 31 of the recess 3) in the height direction H1 of the projection 2. Thus, it is possible to further enhance the effect of holding the projection 2 by the holding portion 5.

Furthermore, in the manufacturing apparatus 10 according to the present embodiment, since ultrasonic vibration is used as a means for forming the hole 4 and the holding portion 5, it is possible to locally melt a portion of the nonwoven fabric S associated with the top surface 16. Therefore, it is possible to maximally avoid that the other portion such as a portion of the projection 2 on the side of the base portion B, and the recess 3 is thermally affected, when the hole 4 is formed in the projection 2. Thus, it is possible to form the holding portion 5 having a high rigidity by cooling and hardening a part of the projection 2 melted by ultrasonic vibration, in the periphery of the hole 4. Further, it is possible to avoid that the rigidity of the portion other than the holding portion 5 is made high, unlike the holding portion 5, and prevent loss of flexibility. When the sheet 1 is used as the sheet 1, in a disposable wearable article, for covering an absorbent member and for use in a portion in contact with the skin, a side of the apex 2A of the projection 2 where the hole 4, and the holding portion 5 having a high rigidity in the periphery of the hole 4 are formed is directed to the absorbent member; and a side of the base portion of the projection 2, or a side of the bottom portion of the recess 3 projecting opposite to the projection 2 is directed to the skin surface of a wearer. Thus, it is possible to speedily guide liquid to the absorbent member and to prevent lowering of a property such as a texture, when the sheet is used as the sheet 1 in a disposable wearable article.

Moreover, in the manufacturing apparatus 10 according to the present embodiment, the adjoining surface 17 has a shape such that a cross-sectional area of the protrusion 15 decreases as a distance from the top surface 16 decreases. Therefore, when a portion of the projection 2 melted by ultrasonic vibration is pressed against the top surface 16, and moved to the periphery of the hole 4, the melted portion is easily moved along the adjoining surface 17. Thus, it is easy to stereoscopically form the holding portion 5 to also spread toward the base portion B of the projection 2 (toward the bottom portion 31 of the recess 3) in the height direction H1 of the projection 2. Therefore, the effect of holding the projection 2 is enhanced to thereby easily maintain the cushioning property of the sheet 1.

In addition, since the manufacturing apparatus 10 according to the present embodiment includes the suction device 20, it is possible to form the hole 4 and the holding portion 5 in the projection 2, while attracting a portion of the nonwoven fabric S between the projections 2 toward the base end 15B of the pin 15 by causing the suction device 20 to suck air. Specifically, during a period after a part of the projection 2 is melted by ultrasonic vibration, and the hole 4 is formed, until the holding portion 5 is cooled and hardened, it is possible to maintain a state that the nonwoven fabric S is pulled by suction. Therefore, it is possible to keep the shape of the projection 2 until the holding portion 5 is hardened. Further, it is possible to prevent that the hole 4 shrinks or the hole 4 closes before the holding portion 5 is hardened. Thus, it is possible to form the hole 4 more clearly.

In the manufacturing apparatus 10 according to the present embodiment, the application surface 13 is a flat surface having a size such that the entirety of the top surface 16 and the entirety of the adjoining surface 17 are included, when the application surface 13 and the protrusion 15 are viewed in the height direction H2 of the protrusion 15 in a state that the top surface 16 of the protrusion 15 on the roller 12 faces the application surface 13 of the ultrasonic horn 11. Therefore, when a part of the projection 2 melted by ultrasonic vibration is pressed against the top surface 16, and moved to the periphery of the hole 4, movement in a direction opposite to the side of the base end 15B of the protrusion 15 is restrained by the application surface 13. Thus, the melted part of the projection 2 is more easily moved along the adjoining surface 17.

In the manufacturing method according to the present embodiment, it is possible to melt a part of the projection 2 and form the hole 4 by applying ultrasonic vibration in a state that the nonwoven fabric S is sandwiched between the application surface 13 and the opposing surface 14. Further, it is possible to form the holding portion 5 by cooling the melted part of the projection 2 in the periphery of the hole 4. Thus, it is possible to form, from a single nonwoven fabric, the sheet 1 for a disposable wearable article in which the projection 2 is held, and an open state of the hole 4 is held.

Further, in the manufacturing method according to the present embodiment, in the projection forming process, the projection 2 and the recess 3 are formed by attracting the nonwoven fabric S to the outer peripheral surface 14 and the outer surfaces of the plurality of protrusions 15 by sucking air through the suction ports 21. Then, also in the hole forming process and the holding portion forming process, air suction through the suction ports 21 is continuously performed. Therefore, it is possible to form the hole 4 and the holding portion 5 in the projection 2 by attracting the nonwoven fabric S to the outer circumferential surface 14 and the pins 15 by sucking air. Specifically, during a period after a part of the projection 2 is melted by ultrasonic vibration, and the hole 4 is formed, until the holding portion 5 is cooled and hardened, it is possible to maintain a state that the nonwoven fabric S is pulled by suction. Therefore, it is possible to keep the shape of the projection 2 until the holding portion 5 is hardened. Further, it is possible to prevent that the hole 4 shrinks or the hole 4 closes before the holding portion 5 is hardened. Thus, it is possible to form the hole 4 more clearly, and it is possible to enhance the liquid permeability of the sheet.

### [Modification]

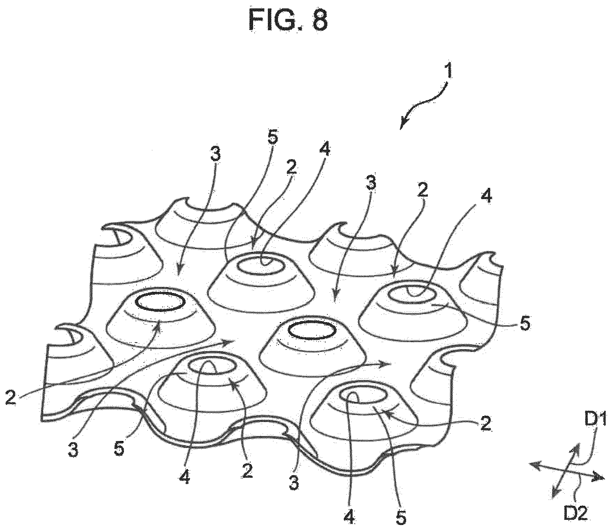

FIG. 8 is a perspective view illustrating a sheet 1 according to a modification of the embodiment. The sheet 1 according to the modification illustrated in FIG. 8 is such that a plurality of projections 2 are formed on a single nonwoven fabric. A hole 4 is formed in an apex 2A of the projection 2. The projection 2 includes a holding portion 5 for holding the projection 2 and holding an open state of the hole 4, in the periphery of the hole 4 and at the position adjacent to the hole 4. The sheet 1 according to the modification is similar to the sheet I according to the embodiment illustrated in FIG. 1 in a point that the sheet 1. has the above-described configuration. In the sheet 1. according to the modification, however, a periphery of each of the projections 2 is constituted of a substantially flat surface. Therefore, in the sheet 1 according to the modification, the plurality of recesses 3 in the sheet 1 according to the embodiment illustrated in FIG. 1 are not clearly formed. The sheet 1 according to the modification is different from the sheet 1 according to the embodiment illustrated in FIG. 1 in this feature.

### [Other Modifications]

In the embodiment, the top surface 16 of the protrusion 15 is, for example, a flat surface. Alternatively, the top surface 16 may be a curved surface. Specifically, a surface of a protrusion 15 on a side of a distal end 15A may have a shape like a part of a spherical surface. Further, the protrusion 15 may have a shape like a frustum shape, for example. In this case, the top surface 16 of the protrusion 15 may be a flat surface or may be a curved surface.

In the embodiment, a shape of the protrusion 15 on a cross section orthogonal to the height direction H2 thereof is a circular shape. The embodiment is not limited to the above. The shape of the cross section may be another shape such as an elliptical shape or a polygonal shape.

In the embodiment, the sheet manufacturing apparatus includes the suction device 20. The suction device 20 can be omitted. In a case that the suction device 20 is omitted, as a method for forming a plurality of projections on a nonwoven fabric S in the sheet manufacturing method, for example, a means for attracting the nonwoven fabric S toward an outer circumferential surface 14 of a roller 12 and base ends 15B of pins 15 by a tension force to be generated when the nonwoven fabric S is wound on the roller 12 of an anvil is exemplified.

Further, in the projection forming process, it is possible to provide an opposing roller including recesses engageable with pins 15 on a roller 12 of an anvil, cause the opposing roller to press against the roller 12, and form projections on a nonwoven fabric S by passing the nonwoven fabric S between the opposing roller and the roller 12. In this case, it is preferable to provide a suction device in order to hold the formed projections. Further, when the opposing roller does not have recesses but has a large number of pins, and the pins are engaged with the pins 15 on the roller 12 of the anvil, a configuration in which projections and recesses are alternately aligned is formed on the nonwoven fabric S.

The above-described specific embodiment mainly includes an invention having the following configuration.

The present invention is directed to a sheet for use in a disposable wearable article. The sheet is such that a plurality of projections are formed on a single nonwoven fabric. A hole is formed in an apex of the projection. The projection includes a holding portion, in a periphery of the hole and at a position adjacent to the hole, for holding the projection and holding an open state of the hole.

In the sheet according to the present invention, the holding portion formed in the periphery of the hole and at the position adjacent to the hole holds the projection and holds the open state of the hole. Specifically, the projection formed on the sheet is held by the holding portion, and can be continued to be present on the sheet. Further, the open state of the hole formed in the sheet is held by the holding portion. Therefore, it is possible to form, from a single nonwoven fabric, a sheet for a disposable wearable article in which a projection is held, and an open state of a hole is held.

In the sheet, preferably, the holding portion may include a part which approaches a base portion of the projection in a height direction of the projection as a distance from the hole in a direction orthogonal to the height direction increases.

In this configuration, the holding portion is not only planarly formed to simply surround the periphery of the hole, but also is stereoscopically formed to spread toward the base portion of the projection in the height direction of the projection. Thus, the effect of holding the projection by the holding portion can be further enhanced.

In the sheet, preferably, a rigidity of the holding portion may be higher than a rigidity of a portion other than the holding portion.

In this configuration, the projection and the open state of the hole are held by the holding portion in which the rigidity is made high, as compared with the portion other than the holding portion.

In the sheet, preferably, a mass per unit area of the holding portion may be greater than a mass per unit area of a portion other than the holding portion.

In this configuration, the projection and the open state of the hole are held by the holding portion in which the mass per unit area is increased, as compared with the portion other than the holding portion.

A sheet manufacturing apparatus according to the present invention is an apparatus for manufacturing a sheet for use in a disposable wearable article from a single nonwoven fabric. The manufacturing apparatus includes: an ultrasonic horn including an application surface for applying ultrasonic vibration; and an anvil including an opposing surface capable of facing the application surface. A plurality of protrusions are formed on the opposing surface. Each of the protrusions includes a top surface located at a distal end of the protrusion, and an adjoining surface that is formed to adjoin the top surface in such a way as to extend toward a base end of the protrusion, while spreading from the top surface to a periphery of the top surface. The manufacturing apparatus is configured to apply ultrasonic vibration with the nonwoven fabric being sandwiched between the application surface and the opposing surface in a state that a plurality of projections are formed on the nonwoven fabric by attracting the nonwoven fabric to the opposing surface where the plurality of protrusions are formed in such a way as to melt a portion of the nonwoven fabric associated with the top surface and form a hole in the projection, and is configured to cool the melted portion in a periphery of the hole in such a way as to form a holding portion, in the periphery of the hole and at a position adjacent to the hole, for holding the projection and holding an open state of the hole.

In the manufacturing apparatus according to the present invention, since the hole and the holding portion are formed by melting the portion of the nonwoven fabric associated with the top surface by ultrasonic vibration, it is possible to allow the holding portion to hold the projection and the open state of the hole. Thus, it is possible to form, from a single nonwoven fabric, a sheet for a disposable wearable article in which a projection is held, and an open state of a hole is held.

Further, in the manufacturing apparatus according to the present invention, each of the protrusions includes the top surface located at the distal end of the protrusion, and the adjoining surface that is formed to adjoin the top surface in such a way as to extend toward the base end of the protrusion, while spreading from the top surface to the periphery thereof. Therefore, when a part of the projection is melted, and the hole is formed by application of ultrasonic vibration in a state that the nonwoven fabric is sandwiched between the application surface and the opposing surface, the melted part is pressed against the top surface. Accordingly, the melted part is moved to the periphery of the hole along the adjoining surface that is formed to adjoin the top surface. The thus moved part is cooled and solidified at a position along the adjoining surface. Consequently, the holding portion is not only planarly formed to simply surround the periphery of the hole, but also is stereoscopically formed to spread toward the base portion of the projection in the height direction of the projection. Thus, it is possible to further enhance the effect of holding the projection by the holding portion.

Furthermore, in the manufacturing apparatus according to the present invention, since ultrasonic vibration is used as a means for forming the hole and the holding portion, it is possible to locally melt the portion of the nonwoven fabric associated with the top surface. Therefore, for example, unlike a case that a plurality of protrusions themselves are heated in advance, and are caused to come into contact with a nonwoven fabric to thereby form a hole, in the manufacturing apparatus according to the present invention, it is possible to maximally avoid that the other portion such as a portion of the projection on the side of the base portion, and the periphery thereof is thermally affected. Thus, it is possible to form the holding portion having a high rigidity by cooling and hardening the part of the projection that is locally melted by ultrasonic vibration, in the periphery of the hole. Further, it is possible to avoid that the rigidity of the other portion increases, unlike the holding portion, and prevent loss of flexibility. Therefore, when the sheet is used as a sheet for a disposable wearable article, it is possible to prevent lowering of a property such as a texture.

In the sheet manufacturing apparatus, preferably, the adjoining surface may have a shape such that, as a distance from the top surface decreases, a cross-sectional area of the protrusion decreases.

In this configuration, since the adjoining surface has the above-described shape (e.g. a shape such as a tapered shape and a curved surface shape), the part melted by ultrasonic vibration is easily moved along the adjoining surface, when being pressed against the top surface, and moved to the periphery of the hole.

The sheet manufacturing apparatus may preferably further include a suction device for sucking air through a suction port formed in a position on the opposing surface between the protrusions adjacent to each other so as to attract the nonwoven fabric to the opposing surface.

In this configuration, it is possible to form the hole and the holding portion in the projection, while attracting the portion of the nonwoven fabric between the projections toward the base ends of the protrusions by causing the suction device to suck air. Therefore, in a case that suction is continued for a period after the part of the projection is melted by ultrasonic vibration, and the hole is formed, until the holding portion is cooled and hardened, it is possible to maintain a state that the nonwoven fabric is pulled by suction until the holding portion is hardened. Thus, it is possible to keep the shape of the projection until the holding portion is hardened. Further, it is possible to prevent that the hole shrinks or the hole closes before the holding portion is hardened. Thus, it is possible to form the hole more clearly.

In the sheet manufacturing apparatus, preferably, the application surface may be a flat surface having a size such that an entirety of the top surface and an entirety of the adjoining surface are included, when the application surface and the protrusion are viewed in a height direction of the protrusion in a state that the top surface of the protrusion faces the application surface.

In this configuration, since the application surface facing the top surface and the adjoining surface of the protrusion is a flat surface having a size such that the entirety of the top surface and the entirety of the adjoining surface are included, when a part of the projection melted by ultrasonic vibration is pressed against the top surface, and moved to the periphery of the hole, movement in a direction opposite to the side of the base end of the protrusion is restrained by the application surface. Thus, the melted part of the projection is more easily moved along the adjoining surface.

A sheet manufacturing method according to the present invention is a method for manufacturing a sheet for use in a disposable wearable article from a single nonwoven fabric. The sheet manufacturing method includes: a projection forming process of forming a plurality of projections on the nonwoven fabric; a hole forming process of melting at least a part of an apex of the projection and forming a hole by application of ultrasonic vibration with the nonwoven fabric being sandwiched between an application surface of an ultrasonic horn for applying ultrasonic vibration and an opposing surface of an anvil facing the application surface in a state that the plurality of projections are formed on the nonwoven fabric; and a holding portion forming process of forming a holding portion, in a periphery of the hole and at a position adjacent to the hole, for holding the projection and holding an open state of the hole by cooling, in the periphery of the hole, at least a part of the apex of the projection melted in the hole forming process.

In the manufacturing method according to the present invention, it is possible to melt at least the part of the apex of the projection and form the hole by applying ultrasonic vibration in a state that the nonwoven fabric is sandwiched between the application surface and the opposing surface. Further, it is possible to form the holding portion by cooling the melted part of the projection in the periphery of the hole. Thus, it is possible to form, from a single nonwoven fabric, a sheet for a disposable wearable article in which a projection is held, and an open state of a hole is held.

In the sheet manufacturing method, preferably, a plurality of protrusions may be formed on the opposing surface, and a suction port may be formed between the protrusions. The projection forming process may include forming the plurality of projections by attracting the nonwoven fabric to the opposing surface by sucking air through the suction port. Each of the hole forming process and the holding portion forming process may further include sucking air through the suction port.

In this configuration, it is possible to form the hole in the apex of the projection, and form the holding portion in the periphery of the hole, while attracting the nonwoven fabric to the opposing surface of the anvil and outer surfaces of the protrusions by sucking air. Specifically, during a period after a part of the projection is melted by ultrasonic vibration, and the hole is formed, until the holding portion is cooled and hardened, it is possible to maintain a state that the nonwoven fabric is pulled by suction. Therefore, it is possible to keep the shape of the projection until the holding portion is hardened. Further, it is possible to prevent that the hole shrinks or the hole closes before the holding portion is hardened. Thus, it is possible to form the hole more clearly, and it is possible to enhance the liquid permeability of the sheet.

## Claims

1. A sheet for use in a disposable wearable article, wherein
the sheet is such that a plurality of projections are formed on a single nonwoven fabric,
a hole is formed in an apex of the projection, and
the projection includes a holding portion, in a periphery of the hole and at a position adjacent to the hole, for holding the projection and holding an open state of the hole.

2. The sheet according to claim 1, wherein
the holding portion includes a part which approaches a base portion of the projection in a height direction of the projection as a distance from the hole in a direction orthogonal to the height direction increases.

3. The sheet according to claim 1 or 2, wherein
a rigidity of the holding portion is higher than a rigidity of a portion other than the holding portion.

4. The sheet according to any one of claims 1 to 3, wherein
a mass per unit area of the holding portion is greater than a mass per unit area of a portion other than the holding portion.

5. A sheet manufacturing apparatus for manufacturing a sheet for use in a disposable wearable article from a single nonwoven fabric, comprising:
an ultrasonic horn including an application surface for applying ultrasonic vibration; and
an anvil including an opposing surface capable of facing the application surface, wherein
a plurality of protrusions are formed on the opposing surface,
each of the protrusions includes a top surface located at a distal end of the protrusion, and an adjoining surface that is formed to adjoin the top surface in such a way as to extend toward a base end of the protrusion, while spreading from the top surface to a periphery of the top surface, and
the manufacturing apparatus is configured to apply ultrasonic vibration with the nonwoven fabric being sandwiched between the application surface and the opposing surface in a state that a plurality of projections are formed on the nonwoven fabric by attracting the nonwoven fabric to the opposing surface where the plurality of protrusions are formed in such a way as to melt a portion of the nonwoven fabric associated with the top surface and form a hole in the projection, and is configured to cool the melted portion in a periphery of the hole in such a way as to form a holding portion, in the periphery of the hole and at a position adjacent to the hole, for holding the projection and holding an open state of the hole.

6. The sheet manufacturing apparatus according to claim 5, wherein
the adjoining surface has a shape such that, as a distance from the top surface decreases, a cross-sectional area of the protrusion decreases.

7. The sheet manufacturing apparatus according to claim 5 or 6, further comprising:
a suction device for sucking air through a suction port formed in a position on the opposing surface between the protrusions adjacent to each other so as to attract the nonwoven fabric to the opposing surface.

8. The sheet manufacturing apparatus according to any one of claims 5 to 7, wherein
the application surface is a flat surface having a size such that an entirety of the top surface and an entirety of the adjoining surface are included, when the application surface and the protrusion are viewed in a height direction of the protrusion in a state that the top surface of the protrusion faces the application surface.

9. A sheet manufacturing method for manufacturing a sheet for use in a disposable wearable article from a single nonwoven fabric, the sheet manufacturing method comprising:
a projection forming process of forming a plurality of projections on the nonwoven fabric;
a hole forming process of melting at least a part of an apex of the projection and forming a hole by application of ultrasonic vibration with the nonwoven fabric being sandwiched between an application surface of an ultrasonic horn for applying ultrasonic vibration and an opposing surface of an anvil facing the application surface in a state that the plurality of projections are formed on the nonwoven fabric; and
a holding portion forming process of forming a holding portion, in a periphery of the hole and at a position adjacent to the hole, for holding the projection and holding an open state of the hole by cooling, in the periphery of the hole, at least a part of the apex of the projection melted in the hole forming process.

10. The sheet manufacturing method according to claim 9, wherein
a plurality of protrusions are formed on the opposing surface, and a suction port is formed between the protrusions,
the projection forming process includes forming the plurality of projections by attracting the nonwoven fabric to the opposing surface by sucking air through the suction port, and
each of the hole forming process and the holding portion forming process further includes sucking air through the suction port.
